# EUROPEAN PATENT SPECIFICATION

(11) **EP 4 231 956 B1**
(45) Date of publication and mention of the grant of the patent: **05.08.2026**
(21) Application number: 21811555.8
(22) Date of filing: 12.10.2021
(51) Int. Cl.: A61B 90/00, A61B 34/30, A61B 34/10, A61B 34/20

(54) **SYSTEMS AND METHODS FOR SEGMENTAL TRACKING**
SYSTEME UND VERFAHREN ZUR SEGMENTBASIERTEN VERFOLGUNG
SYSTÈMES ET PROCÉDÉS DE SUIVI SEGMENTAIRE

(30) Priority: 22.10.2020 US 202063104303 P; 29.09.2021 US 202117489498
(43) Date of publication of application: 30.08.2023
(73) Proprietor: Medtronic Navigation, Inc., Louisville CO 80027 (US)
(72) Inventor: WALD, Andrew J., Louisville, Colorado 80027 (US); SNYDER, Victor D., Louisville, Colorado 80027 (US); RONEN, Shai, Louisville, Colorado 80027 (US); MAHENDRA, Nikhil, Louisville, Colorado 80027 (US); SINHA, Shiva R., Louisville, Colorado 80027 (US); JACOBSEN, Bradley W., Louisville, Colorado 80027 (US); HARTMANN, Steven, Louisville, Colorado 80027 (US); BHARADWAJ, Jeetendra S., Louisville, Colorado 80027 (US)
(74) Representative: Viering, Jentschura & Partner mbB Patent- und Rechtsanwälte
(86) International application number: PCT/US2021/054581
(87) International publication number: WO 2022/086760

(56) References cited:
- WO-A1-2011/134083
- US-A1- 2020 237 444

## Description

### FIELD

The present technology generally relates to surgical imaging and navigation, and relates more particularly to tracking anatomical elements during surgery.

### BACKGROUND

Surgical navigation systems are used to track the position of one or more objects during surgery. Surgical imaging systems may be used to obtain preoperative, intraoperative, and/or postoperative images. Surgical procedures (or portions thereof) may be planned based on a position of an anatomical element shown in preoperative and/or intraoperative images.

According to WO 2011/134083 A1, systems and methods for surgical guidance and image registration are provided, in which three-dimensional image data associated with an object or patient is registered to topological image data obtained using a surface topology imaging device. The surface topology imaging device may include fiducial markers, which may be tracked by an optical position measurement system that also tracks fiducial markers on a movable instrument. The instrument may be registered to the topological image data, such that the topological image data and the movable instrument are registered to the three-dimensional image data. The three-dimensional image data may be CT or MRI data associated with a patient. The system may also co-register images pertaining to a surgical plan with the three-dimensional image data. In another aspect, the surface topology imaging device may be configured to directly track fiducial markers on a movable instrument. The fiducial markers may be tracked according to surface texture.

US 2020/237444 A1 discloses a system for assisting in guiding and performing a procedure on a subject. The subject may be any appropriate subject such as inanimate object and/or an animate object. The guide and system may include various manipulable or movable members and may be registered to selected coordinate systems.

### SUMMARY

The invention provides a segmental tracking method according to claim 1, and a segmental tracking system according to claim 10. Further embodiments are described in the dependent claims.

It is to be appreciated that any feature described herein can be claimed in combination with any other feature(s) as described herein, regardless of whether the features come from the same described embodiment.

The details of one or more aspects of the disclosure are set forth in the accompanying drawings and the description below. Other features, objects, and advantages of the techniques described in this disclosure will be apparent from the description and drawings, and from the claims.

The details of one or more aspects of the disclosure are set forth in the accompanying drawings and the description below. Other features, objects, and advantages of the techniques described in this disclosure will be apparent from the description and drawings, and from the claims.

The phrases "at least one", "one or more", and "and/or" are open-ended expressions that are both conjunctive and disjunctive in operation. For example, each of the expressions "at least one of A, B and C", "at least one of A, B, or C", "one or more of A, B, and C", "one or more of A, B, or C" and "A, B, and/or C" means A alone, B alone, C alone, A and B together, A and C together, B and C together, or A, B and C together. When each one of A, B, and C in the above expressions refers to an element, such as X, Y, and Z, or class of elements, such as X₁-Xₙ, Y₁-Yₘ, and Z₁-Zₒ, the phrase is intended to refer to a single element selected from X, Y, and Z, a combination of elements selected from the same class (e.g., X₁ and X₂) as well as a combination of elements selected from two or more classes (e.g., Y₁ and Zₒ).

The term "a" or "an" entity refers to one or more of that entity. As such, the terms "a" (or "an"), "one or more" and "at least one" can be used interchangeably herein. It is also to be noted that the terms "comprising", "including", and "having" can be used interchangeably.

The preceding is a simplified summary of the disclosure to provide an understanding of some aspects of the disclosure. This summary is neither an extensive nor exhaustive overview of the disclosure and its various aspects, embodiments, and configurations. It is intended neither to identify key or critical elements of the disclosure nor to delineate the scope of the disclosure but to present selected concepts of the disclosure in a simplified form as an introduction to the more detailed description presented below. As will be appreciated, other aspects, embodiments, and configurations of the disclosure are possible utilizing, alone or in combination, one or more of the features set forth above or described in detail below.

Numerous additional features and advantages of the present invention will become apparent to those skilled in the art upon consideration of the embodiment descriptions provided hereinbelow.

### BRIEF DESCRIPTION OF THE DRAWINGS

The accompanying drawings are incorporated into and form a part of the specification to illustrate several examples of the present disclosure. These drawings, together with the description, explain the principles of the disclosure. The drawings simply illustrate preferred and alternative examples of how the disclosure can be made and used and are not to be construed as limiting the disclosure to only the illustrated and described examples. Further features and advantages will become apparent from the following, more detailed, description of the various aspects, embodiments, and configurations of the disclosure, as illustrated by the drawings referenced below.
Fig. 1 is a block diagram of a system according to at least one embodiment of the present disclosure;
Fig. 2 is a flowchart of a method according to at least one embodiment of the present disclosure;
Fig. 3 is a flowchart of another method according to at least one embodiment of the present disclosure;
Fig. 4A is a flowchart of another method according to at least one embodiment of the present disclosure;
Fig. 4B is a block diagram of a passive fiducial marker according to at least one embodiment of the present disclosure;
Fig. 4C is a block diagram of an active fiducial marker according to at least one embodiment of the present disclosure; and
Fig. 5 is a flowchart of another method according to at least one embodiment of the present disclosure.

### DETAILED DESCRIPTION

It should be understood that various aspects disclosed herein may be combined in different combinations than the combinations specifically presented in the description and accompanying drawings. It should also be understood that, depending on the example or embodiment, certain acts or events of any of the processes or methods described herein may be performed in a different sequence, and/or may be added, merged, or left out altogether (e.g., all described acts or events may not be necessary to carry out the disclosed techniques according to different embodiments of the present disclosure). In addition, while certain aspects of this disclosure are described as being performed by a single module or unit for purposes of clarity, it should be understood that the techniques of this disclosure may be performed by a combination of units or modules associated with, for example, a computing device and/or a medical device.

In one or more examples, the described methods, processes, and techniques may be implemented in hardware, software, firmware, or any combination thereof. If implemented in software, the functions may be stored as one or more instructions or code on a computer-readable medium and executed by a hardware-based processing unit. Computer-readable media may include non-transitory computer-readable media, which corresponds to a tangible medium such as data storage media (e.g., RAM, ROM, EEPROM, flash memory, or any other medium that can be used to store desired program code in the form of instructions or data structures and that can be accessed by a computer).

Instructions may be executed by one or more processors, such as one or more digital signal processors (DSPs), general purpose microprocessors (e.g., Intel Core i3, i5, i7, or i9 processors; Intel Celeron processors; Intel Xeon processors; Intel Pentium processors; AMD Ryzen processors; AMD Athlon processors; AMD Phenom processors; Apple A10 or 10X Fusion processors; Apple A11, A12, A12X, A12Z, or A13 Bionic processors; or any other general purpose microprocessors), application specific integrated circuits (ASICs), field programmable logic arrays (FPGAs), or other equivalent integrated or discrete logic circuitry. Accordingly, the term "processor" as used herein may refer to any of the foregoing structure or any other physical structure suitable for implementation of the described techniques. Also, the techniques could be fully implemented in one or more circuits or logic elements.

Before any embodiments of the disclosure are explained in detail, it is to be understood that the disclosure is not limited in its application to the details of construction and the arrangement of components set forth in the following description or illustrated in the drawings. The disclosure is capable of other embodiments and of being practiced or of being carried out in various ways. Also, it is to be understood that the phraseology and terminology used herein is for the purpose of description and should not be regarded as limiting. The use of "including," "comprising," or "having" and variations thereof herein is meant to encompass the items listed thereafter and equivalents thereof as well as additional items. Further, the present disclosure may use examples to illustrate one or more aspects thereof. Unless explicitly stated otherwise, the use or listing of one or more examples (which may be denoted by "for example," "by way of example," "e.g.," "such as," or similar language) is not intended to and does not limit the scope of the present disclosure.

Segmental tracking refers to the tracking of individual anatomical elements, such as the one or more vertebrae of a spine. Segmental tracking may be used, for example, to enable adjustment of any preoperative plan based on movement (whether planned or unexpected) of the tracked anatomical element(s) during surgery. The terms object tracking, element tracking, and anatomical tracking may be used herein as synonyms for segmental tracking.

Spinal navigation and robotics tend to assume that the anatomy of interest remains fixed after registration. In practice, the relative positions of the vertebrae may change during surgery, and if the change is sufficiently significant, re-registration may be required. Segmental tracking resolves this problem by tracking individual vertebrae in real-time or near real-time and updating the clinical images and/or digital models accordingly.

Segmental tracking may be used to quantify whether and/or to what extent surgical goals were achieved. For example, anatomy correction is the crux of many spine procedures, and segmental tracking may be used to quantify the amount of anatomy correction achieved. This is possible because segmental tracking enables the position and orientation of each individual vertebral level to be known in physical space.

Embodiments of the present disclosure have the potential to enable more accurate navigation as the anatomy can move freely (which movement is detected by the segmental tracking and can therefore be accounted for by the navigation system) and is not constrained by a single, fixed registration. When segmental tracking is implemented via ultrasound or radar imaging of the individual vertebral levels, the surgical workflow becomes more efficient, less costly, and less invasive.

Some embodiments of the present disclosure utilize topographic or tomographic systems and/or methods to image individual vertebral levels in real-time or near real-time and provide enough surface or feature resolution to be registered to other medical imaging modalities (including, for example, a preoperative CT scan or MRI image). Ultrasound may be used to create a model of the bone/soft tissue interface (comprising surface data, volumetric data, or both) without invasive measures. Similarly, radar may be used to create a model of the bone/soft tissue interface (again comprising surface data, volumetric data, or both) without invasive measures. When ultrasound and/or radar are applied as described herein to surgery (including, for example, spine surgery), ultrasound and/or radar data may be registered to preoperative medical imaging data to help surgeons execute and monitor the amount of correction they aim to achieve during the course of their procedure. More particularly, the use of ultrasound and radar technologies may be used to obtain surface, volumetric, and/or feature information that can be used to find continuous registration transforms of individual vertebral levels mapped to image space as the levels move during a spine surgery. Embodiments of the present disclosure may be used in a similar manner to enable anatomical tracking during other surgeries. In other words, the present disclosure is not limited to spinal surgery applications.

Directly imaging the anatomy to provide position and orientation information advantageously relieves clinicians of the burden of creating additional access to the anatomy to affix a fiducial or other tracking device. Similarly, embodiments of the present disclosure have the potential to reduce system complexity, disposable costs, and ultimately provide a better solution for customers that are interested in quantifying the correction they achieve during surgically navigated or robotic spine procedures.

Some embodiments of the present disclosure encompass, among other concepts, the use of ultrasound to create multiple reference images to compute per-level registration against another medical imaging data set; the use of radar to create multiple reference images to compute per-level registration against another medical imaging data set; and the use of these imaging technologies as part of a segmental tracking solution wherein individual vertebral bodies are tracked relative to each other and tools/instruments/implants are tracked relative to the individual levels without the use of fiducial markers placed directly on the anatomy.

In some embodiments of the present disclosure, ultrasonic fiducials are attached to each anatomical element of interest at a surgical site (e.g., each vertebra affected by a spinal surgery), associated with the corresponding parts of the exam volume (e.g., with the corresponding anatomical element in preoperative or intraoperative imaging and/or in a model of the anatomic volume of interest), and continuously localized. The information may be used, for example, to assess anatomical correction intraoperatively, and to allow accurate tool navigation with respect to dynamic anatomy.

The use of ultrasonic fiducial markers (rather than fiducial markers intended for detection using X-ray-based imaging techniques) beneficially reduces the level of radiation exposure experienced by those within an operating room during a surgery that uses such ultrasonic fiducial markers. Fiducial markers intended and/or optimized for use with other imaging modalities that do not use X-rays, such as radar, yield the same benefit.

Embodiments of the present disclosure provide technical solutions to one or more of the problems of (1) tracking motion of anatomical elements during a surgery, including unintended and/or undesired motion; (2) avoiding the need for re-registration due to the intraoperative movement of one or more anatomical elements; (3) reducing or eliminating the need for invasive procedures to secure fiducials to anatomical elements to be tracked using a segmental tracking system; (4) reducing or eliminating a need to utilize X-ray-based imaging, or other imaging that exposes the patient and/or operating room staff to potentially harmful radiation, for segmental tracking; (5) simplifying the surgical workflow and thus reducing the workload of surgeons and other operating room staff; (6) eliminating steps from the surgical workflow with resulting savings of time, money, and other resources; and (7) improving the accuracy of navigation and/or robotic guidance by ensuring that such guidance is generated based on an actual position of relevant anatomical elements.

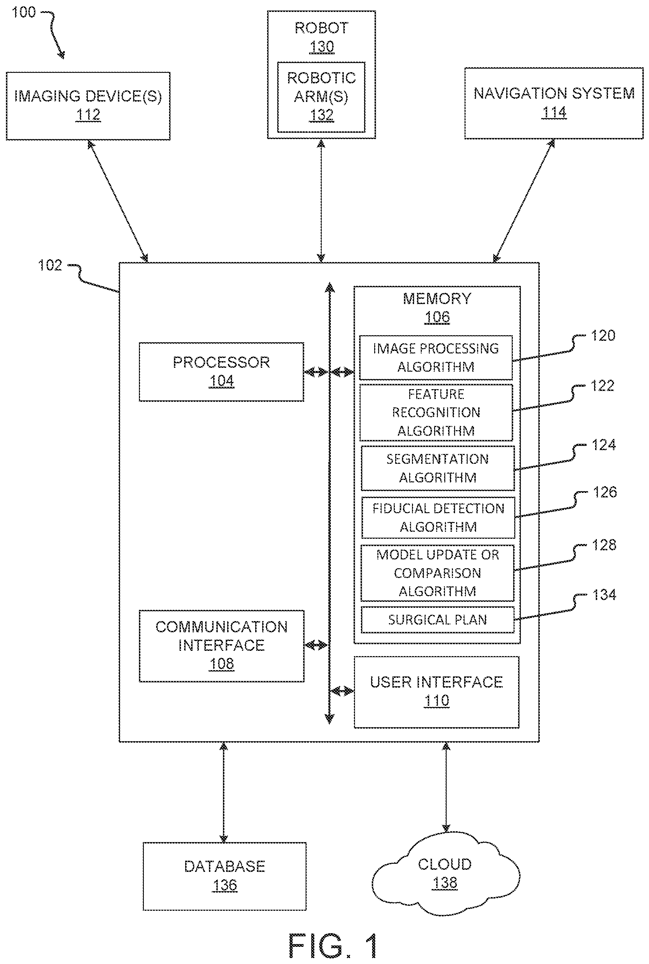

Turning first to Fig. 1, a block diagram of a system 100 according to at least one embodiment of the present disclosure is shown. The system 100 may be used to obtain and process image data (e.g., in connection with segmental tracking); execute one or more of the methods described herein; execute an image processing algorithm, a pose algorithm, a registration algorithm, an image update or comparison algorithm, and/or a model update or comparison algorithm; and/or carry out one or more other aspects of one or more of the methods disclosed herein. The system 100 comprises a computing device 102, one or more imaging devices 112, a navigation system 114, a robot 130, a database 136, and/or a cloud 138. Systems according to other embodiments of the present disclosure may comprise more or fewer components than the system 100. For example, the system 100 may not include the navigation system 114, the robot 130, one or more components of the computing device 102, the database 136, and/or the cloud 138.

The computing device 102 comprises a processor 104, a memory 106, a communication interface 108, and a user interface 110. Computing devices according to other embodiments of the present disclosure may comprise more or fewer components than the computing device 102.

The processor 104 of the computing device 102 may be any processor described herein or any similar processor. The processor 104 may be configured to execute instructions stored in the memory 106, which instructions may cause the processor 104 to carry out one or more computing steps utilizing or based on data received from the imaging device 112, the robot 130, the navigation system 114, the database 136, and/or the cloud 138.

The memory 106 may be or comprise RAM, DRAM, SDRAM, other solid-state memory, any memory described herein, or any other tangible, non-transitory memory for storing computer-readable data and/or instructions. The memory 106 may store information or data useful for completing, for example, any step of the methods 200, 300, 400, and/or 500 described herein, or of any other methods. The memory 106 may store, for example, one or more image processing algorithms 120, one or more feature recognition algorithms 122, one or more segmentation algorithms 124, one or more fiducial detection algorithms 126, one or more model update or comparison algorithm 128, and/or one or more surgical plans 134. Such instructions or algorithms may, in some embodiments, be organized into one or more applications, modules, packages, layers, or engines. The algorithms and/or instructions may cause the processor 104 to manipulate data stored in the memory 106 and/or received from or via the imaging device 112, the robot 130, the database 136, and/or the cloud 138.

The computing device 102 may also comprise a communication interface 108. The communication interface 108 may be used for receiving image data or other information from an external source (such as the imaging device 112, the navigation system 114, the robot 130, the database 136, and/or the cloud 138), and/or for transmitting instructions, images, or other information to an external system or device (e.g., another computing device 102, the navigation system 114, the imaging device 112, the robot 130, the database 136, and/or the cloud 138). The communication interface 108 may comprise one or more wired interfaces (e.g., a USB port, an ethernet port, a Firewire port) and/or one or more wireless transceivers or interfaces (configured, for example, to transmit and/or receive information via one or more wireless communication protocols such as 802.11a/b/g/n, Bluetooth, NFC, ZigBee, and so forth). In some embodiments, the communication interface 108 may be useful for enabling the device 102 to communicate with one or more other processors 104 or computing devices 102, whether to reduce the time needed to accomplish a computing-intensive task or for any other reason.

The computing device 102 may also comprise one or more user interfaces 110. The user interface 110 may be or comprise a keyboard, mouse, trackball, monitor, television, screen, touchscreen, and/or any other device for receiving information from a user and/or for providing information to a user. The user interface 110 may be used, for example, to receive a user selection or other user input regarding receiving image data, one or more images, and/or one or more 3D models; to receive a user selection or other user input regarding a surgical plan; to receive a user selection or other user input regarding correlating a representation of an anatomical object in second image data with a representation of the anatomical object in first image data; to receive a user selection or other user input regarding measuring an anatomical angle based on an updated digital model, and/or regarding comparing the measured anatomical angle to a target anatomical angle; to receive a user selection or other user input regarding determining at least one setting of an imaging device 112; to receive a user selection or other user input regarding calculating one or more poses of the imaging device 112; to receive a user selection or other user input regarding correlating at least one fiducial marker to an anatomical object; to receive a user selection or other user input regarding assessing a degree of anatomical correction by comparing a measured anatomical parameter to a planned anatomical parameter; to display a model of an anatomical object; to display a surgical plan; and/or to display a measured and/or a target anatomical parameter. Notwithstanding the foregoing, each of the preceding inputs may be generated automatically by the system 100 (e.g., by the processor 104 or another component of the system 100) or received by the system 100 from a source external to the system 100. In some embodiments, the user interface 110 may be useful to allow a surgeon or other user to modify instructions to be executed by the processor 104 according to one or more embodiments of the present disclosure, and/or to modify or adjust a setting of other information displayed on the user interface 110 or corresponding thereto.

Although the user interface 110 is shown as part of the computing device 102, in some embodiments, the computing device 102 may utilize a user interface 110 that is housed separately from one or more remaining components of the computing device 102. In some embodiments, the user interface 110 may be located proximate one or more other components of the computing device 102, while in other embodiments, the user interface 110 may be located remotely from one or more other components of the computer device 102.

The imaging device 112 may be operable to image anatomical feature(s) (e.g., a bone, veins, tissue, etc.) and/or other aspects of patient anatomy to yield image data (e.g., image data depicting or corresponding to a bone, veins, tissue, etc.). The image data may be first image data comprising pre-operative image data in some examples or post-registration image data in other examples, or second image data obtained intra-operatively in still other examples. In some embodiments, a first imaging device 112 may be used to obtain some image data (e.g., the first image data), and a second imaging device 112-utilizing a different imaging modality than the first imaging device 112-may be used to obtain other image data (e.g., the second image data). The imaging device 112 may be capable of taking a 2D image or a 3D image to yield the image data. "Image data" as used herein refers to the data generated or captured by an imaging device 112, including in a machine-readable form, a graphical/visual form, and in any other form. In various examples, the image data may comprise data corresponding to an anatomical feature of a patient, or to a portion thereof. The imaging device 112 may be or comprise, for example, an ultrasound scanner (which may comprise, for example, a physically separate transducer and receiver, or a single ultrasound probe), a radar system (which may comprise, for example, a transmitter, a receiver, a processor, and one or more antennae), an O-arm, a C-arm, a G-arm, or any other device utilizing X-ray-based imaging (e.g., a fluoroscope, a CT scanner, or other X-ray machine), a magnetic resonance imaging (MRI) scanner, an optical coherence tomography scanner, an endoscope, a telescope, a thermographic camera (e.g., an infrared camera), or any other imaging device 112 suitable for obtaining images of an anatomical feature of a patient.

The imaging device 112 may additionally or alternatively be operable to image the anatomical feature to yield additional image data. The additional image data (which may be, for example, second image data or updated image data) may be obtained in real-time (e.g., with a delay of 500 milliseconds or less, or a delay of 250 milliseconds or less) or near real-time (e.g., with delay of one minute or less, or thirty seconds or less, or ten seconds or less, or five seconds or less, or one second or less). The additional image data may be utilized in conjunction with previously obtained image data (e.g., first image data) for segmental tracking purposes. For example, a representation of an anatomical element in later-obtained image data may be compared to an anatomical element in earlier-obtained image data to detect movement of the anatomical element in the intervening time period. The comparing may comprise correlating the representation of the anatomical element in the second image data to the representation of the anatomical element in the first image data. Such correlating may utilize, for example, one or more of an image processing algorithm 120, a feature recognition algorithm 122, and/or a fiducial detection algorithm 126. The fiducial detection algorithm 126 may enable detection of a representation of a fiducial marker in image data generated by the imaging device 112, and/or may enable determination of a position of the fiducial marker using triangulation and/or other known localization methods. The correlating may ensure that the same anatomical element (having, for example, the same boundaries) is identified in both the first image data and the second image data, so that the comparison is an accurate comparison.

In some embodiments, the imaging device 112 may comprise more than one imaging device 112. For example, a first imaging device may provide first image data and/or a first image set, and a second imaging device may provide second image data and/or a second image set. In still other embodiments, the same imaging device may be used to provide both the first image data and the second image data, and/or any other image data described herein. The imaging device 112 may be operable to generate a stream of image data. For example, the imaging device 112 may be configured to operate with an open shutter, or with a shutter that continuously alternates between open and shut so as to capture successive images. For purposes of the present disclosure, unless specified otherwise, image data may be considered to be continuous and/or provided as an image data stream if the image data represents two or more frames per second.

The navigation system 114 may provide navigation for a surgeon and/or a surgical robot during an operation. The navigation system 114 may be any now-known or future-developed navigation system, including, for example, the Medtronic StealthStation^{™} S8 surgical navigation system or any successor thereof. The navigation system 114 may include a camera or other sensor(s) for tracking one or more reference markers, navigated trackers, or other objects within the operating room or other room in which some or all of the system 100 is located. In various embodiments, the navigation system 114 may be used to track a position and orientation (i.e., pose) of the imaging device 112, the robot 130 and/or robotic arm 132, and/or one or more surgical tools (or, more particularly, to track a pose of a navigated tracker attached, directly or indirectly, in fixed relation to the one or more of the foregoing). The navigation system 114 may include a display for displaying one or more images from an external source (e.g., the computing device 102, imaging device 112, or other source) or for displaying an image and/or video stream from the camera or other sensor of the navigation system 114. In some embodiments, the system 100 can operate without the use of the navigation system 114. The navigation system 114 may be configured to provide guidance to a surgeon or other user of the system 100 or a component thereof, to the robot 130, or to any other element of the system 100 regarding, for example, a pose of one or more anatomical elements, and/or whether or not a tool is in the proper trajectory (and/or how to move a tool into the proper trajectory) to carry out a surgical task according to a preoperative plan.

The robot 130 may be any surgical robot or surgical robotic system. The robot 130 may be or comprise, for example, the Mazor X^{™} Stealth Edition robotic guidance system. The robot 130 may be configured to position the imaging device 112 at one or more precise position(s) and orientation(s), and/or to return the imaging device 112 to the same position(s) and orientation(s) at a later point in time. The robot 130 may additionally or alternatively be configured to manipulate a surgical tool (whether based on guidance from the navigation system 114 or not) to accomplish or to assist with a surgical task. The robot 130 may comprise one or more robotic arms 132. In some embodiments, the robotic arm 132 may comprise a first robotic arm and a second robotic arm, though the robot 130 may comprise more than two robotic arms. In some embodiments, one or more of the robotic arms 132 may be used to hold and/or maneuver the imaging device 112. In embodiments where the imaging device 112 comprises two or more physically separate components (e.g., a transmitter and receiver), one robotic arm 132 may hold one such component, and another robotic arm 132 may hold another such component. Each robotic arm 132 may be positionable independently of the other robotic arm.

The robot 130, together with the robotic arm 132, may have, for example, at least five degrees of freedom. In some embodiments the robotic arm 132 has at least six degrees of freedom. In yet other embodiments, the robotic arm 132 may have less than five degrees of freedom. Further, the robotic arm 132 may be positioned or positionable in any pose, plane, and/or focal point. The pose includes a position and an orientation. As a result, an imaging device 112, surgical tool, or other object held by the robot 130 (or, more specifically, by the robotic arm 132) may be precisely positionable in one or more needed and specific positions and orientations.

In some embodiments, reference markers (i.e., navigation markers) may be placed on the robot 130 (including, e.g., on the robotic arm 132), the imaging device 112, or any other object in the surgical space. The reference markers may be tracked by the navigation system 114, and the results of the tracking may be used by the robot 130 and/or by an operator of the system 100 or any component thereof. In some embodiments, the navigation system 114 can be used to track other components of the system (e.g., imaging device 112) and the system can operate without the use of the robot 130 (e.g., with the surgeon manually manipulating the imaging device 112 and/or one or more surgical tools, based on information and/or instructions generated by the navigation system 114, for example).

The system 100 or similar systems may be used, for example, to carry out one or more aspects of any of the methods 200, 300, 400, and/or 500 described herein. The system 100 or similar systems may also be used for other purposes. In some embodiments, a system 100 may be used to generate and/or display a 3D model of an anatomical feature or an anatomical volume of a patient. For example, the robotic arm 132 (controlled by a processor of the robot 130, the processor 104 of the computing device 102, or some other processor, with or without any manual input) may be used to position the imaging device 112 at a plurality of predetermined, known poses, so that the imaging device 112 can obtain one or more images at each of the predetermined, known poses. Because the pose from which each image is taken is known, the resulting images may be assembled together to form or reconstruct a 3D model. The system 100 may update the model based on information (e.g., segmental tracking information) received from the imaging device 112, as described elsewhere herein.

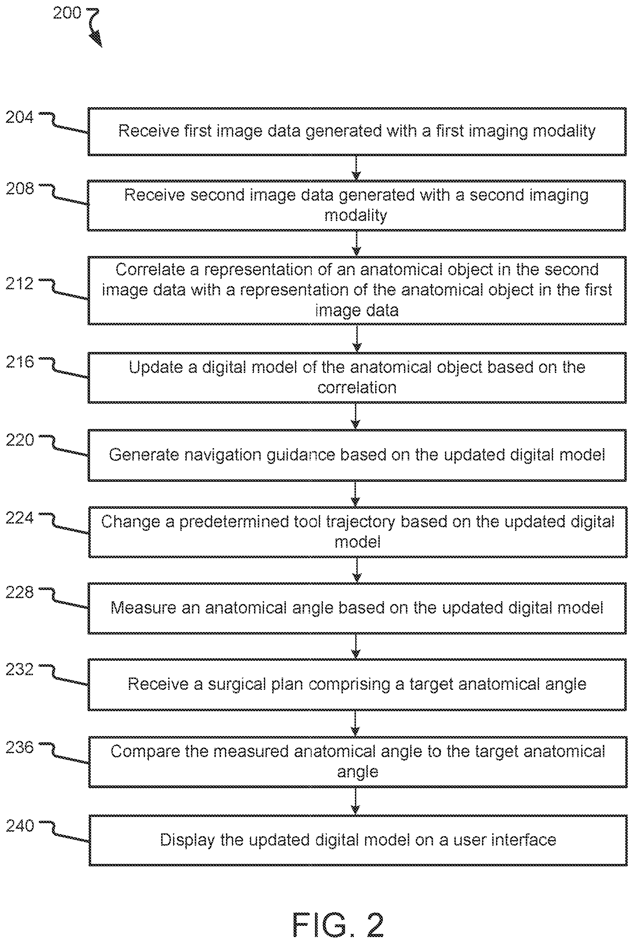

Turning now to Fig. 2, embodiments of the present disclosure may be used, for example, for segmental tracking during a surgical procedure. The surgical procedure may be a spinal surgery or any other surgical procedure.

Fig. 2 depicts a segmental tracking method 200. The segmental tracking method 200 (and/or one or more steps thereof) may be carried out or otherwise performed, for example, by at least one processor. The at least one processor may be the same as or similar to the processor(s) 104 of the computing device 102 described above. The at least one processor may be part of a robot (such as a robot 130) or part of a navigation system (such as a navigation system 114). A processor other than any processor described herein may also be used to execute the method 200. The at least one processor may perform the method 200 by executing instructions stored in a memory such as the memory 106. The instructions may correspond to one or more steps of the method 200 described below. The instructions may cause the processor to execute one or more algorithms, such as the image processing algorithm 120, the feature recognition algorithm 122, the segmentation algorithm 124, the fiducial detection algorithm 126, and/or the model update or comparison algorithm 128.

The method 200 comprises receiving first image data generated with a first imaging modality (step 204). The first image data may be or comprise topographic image data, tomographic image data, or another kind of image data. The first image data may be received, for example, as part of a preoperative plan, and may be or comprise a preoperative image, such as a CT image or an MRI image. In some embodiments, the first image data may be obtained after completion of a registration process that correlates a patient-centric coordinate space to one or both of a navigation system coordinate space and/or a robotic coordinate space. The first image data may correspond to a planned or actual surgical site of a patient, and may comprise a representation of at least one anatomical object of the patient. The at least one anatomical object may be, for example, one or more vertebrae of a spine of the patient (where the patient will undergo spinal surgery), or one or more anatomical elements of the patient's knee (where the patient will undergo knee surgery). The present disclosure is not limited to use in connection with spinal surgery and/or knee surgery, however, and may be used in connection with any surgical procedure.

The first image data may be received, whether directly or indirectly, from an imaging device such as the imaging device 112. The imaging device may be a CT scanner, a magnetic resonance imaging (MRI) scanner, an optical coherence tomography (OCT) scanner, an O-arm (including, for example, an O-arm 2D long film scanner), a C-arm, a G-arm, another device utilizing X-ray-based imaging (e.g., a fluoroscope or other X-ray machine), or any other imaging device. Relatedly, the first imaging modality may be, for example, CT, MRI, OCT, X-ray, or another imaging modality. The first image data may be or comprise one or more two-dimensional (2D) images, and/or one or more three-dimensional (3D) images. In some embodiments, the first image data may be or comprise a 3D model of one or more anatomical elements, which may in turn have been generated using one or more 2D or 3D images.

The method 200 also comprises receiving second image data generated with a second imaging modality (step 208). The second imaging modality may be different that the first imaging modality. The second image data may be or comprise topographic image data, tomographic image data, or another kind of image data. The second image data is received during a surgical procedure, and the second image data may be obtained from an imaging device such as the imaging device 112 during the surgical procedure. The second image data may be received in real-time (e.g., with a delay of 500 milliseconds or less, or a delay of 250 milliseconds or less) or in near real-time (e.g., within one minute or less, or thirty seconds or less, or ten seconds or less, or five seconds or less, or one second or less, after the second image data is generated). The second image data is obtained after the first image data is obtained, and generally corresponds to the same anatomical area as the first image data, or a portion thereof. Thus, for example, if the first image data corresponds to a spine or segment thereof of the patient, then the second image data also corresponds to the spine or segment thereof. As another example, if the first image data corresponds to a knee or portion thereof of the patient, the second image data also corresponds to the knee or a portion thereof. As a result, the second image data comprises a representation of the same at least one anatomical element as the first image data.

The second image data may be received, whether directly or indirectly, from an imaging device such as the imaging device 112. In the method 200, the imaging device utilizes either ultrasound or radar to generate the second image data. The imaging device may be an ultrasound probe comprising a transducer and a receiver in a common housing, or an ultrasound device comprising a transducer and a receiver that are physically separate. Alternatively, the imaging device may be a radar system comprising, for example, a transmitter (including a transmitting antenna) and receiver (including a receiving antenna). The transmitter and the receiver may be contained within a common housing, or may be provided in physically separate housings.

The imaging device that provides the second image data may be fixed in position during an entirety of a surgical task or procedure, and may be configured to generate second image data during the entirety of the surgical task or procedure. In other words, the imaging device may generate a stream of second image data that enables continuous repetition of one or more steps of the method 200 so as to enable continuous updating of a digital model of the surgical site of the patient and facilitate the generation of accurate navigation guidance based on a real-time or near real-time position of the anatomical element. The digital model may be or be comprised within the first image data, or the digital model may have been generated based at least in part on the first image data. In some embodiments, the digital model may not be related to the first image data other than by virtue of being a model of the same (or at least some of the same) anatomical features as are represented in the first image data.

The method 200 also comprises correlating a representation of an anatomical object in the second image data with a representation of the anatomical object in the first image data (step 212). The correlating may comprise and/or correspond to, for example, registering an image generating using the second image data with an image generating using the first image data. The correlating may comprise overlaying a second image generated using the second image data on an first image generated using the first image data, and positioning the second image (including by translation and/or rotation) so that a representation of the anatomical object in the second image aligns with a representation of the anatomical object in the first image. The correlating may occur with or without the use of images generated using the first and/or second image data. In some embodiments, the correlating comprises identifying coordinates of a particular point on the anatomical object in the first image data, and identifying coordinates of the same point on the anatomical object in the second image data (or vice versa). This process may be repeated any number of times for any number of points. The resulting coordinates may be used to determine a transform or offset between the first image data and the second image data, with which any coordinates corresponding to a particular point of an anatomical object as represented in the second image data may be calculated using any coordinates corresponding to the same point of the anatomical object as represented in the first image data, or vice versa.

Any known image registration method or technique may be used to accomplish the correlating. The correlating may comprise utilizing one or more algorithms, such as an image processing algorithm 120, a feature recognition algorithm 122, and/or a segmentation algorithm 124, to identify one or more objects in the first image data and/or in the second image data, including the anatomical object. The one or more algorithms may be algorithms useful for analyzing grayscale image data. In some embodiments, the one or more algorithms may be configured to optimize mutual information in the first image data and the second image data (e.g., to identify a "best fit" between the two sets of image data that causes features in each set of image data to overlap). In some embodiments, a feature recognition algorithm such as the algorithm 122 may utilize edge detection techniques to detect edges between adjacent objects, and thus to define the boundaries of one or more objects. Also in some embodiments, a segmentation algorithm such as the segmentation algorithm 124 may utilize the first image data and/or the second image data to detect boundaries between one or more objects represented in the image data. Where the first image data and/or the second image data comprise topographic and/or tomographic image data, one or more algorithms may be used to analyze the topographic and/or tomographic image data to detect boundaries between, and/or to otherwise segment, one or more anatomical objects represented by or within the image data.

The result of the step 212 is that a representation of an anatomical object in the second image data is matched with a representation of the same anatomical object in the first image data. The anatomical object may be, for example, a vertebra of a spine. Because the vertebra may have moved between a first time when the first image data was generated and a second time when the second image data was generated, the correlating ensures that the same vertebra is identified in both the first image data and the second image data.

The method 200 also comprises updating a digital model of the anatomical object based on the correlation (step 216). The digital model may be, for example, a model to which a navigation system and/or a robotic system is registered or otherwise correlated. The digital model may be based on or obtained from a surgical or other preoperative plan. The digital model may have been generated using, or may otherwise be based on, the first image data. The digital model may be a two-dimensional model or a three-dimensional model. The digital model may be a model of just the anatomical object, or the digital model may be a model of a plurality of anatomical features including the anatomical object. Thus, for example, where the anatomical object is a vertebra, the digital model may be a model of just the vertebra, or the digital model may be a model of a portion or an entirety of the spine, comprising a plurality of vertebrae.

The updating may utilize one or more algorithms, such as the model update or comparison algorithm 128. Where the anatomical object has moved (relative, for example, to one or more other anatomical features represented in the first and/or second image data, to an implant, and/or to a defined coordinate system) from the time at which the first image data was generated to the time at which the second image data was generated, the updating of the digital model of the anatomical object based on the correlation may comprise updating a position of the anatomical object in the digital model relative to one or more other anatomical features in the digital model, relative to the implant, and/or relative to the defined coordinate system. Where the anatomical object has not moved from the time at which the first image data was generated to the time at which the second image data was generated, then updating the digital model may not comprise updating a position of the anatomical object in the digital model.

Independent of whether the anatomical object has moved, the updating may additionally or alternatively comprise adding or updating one or more additional details to the digital model. For example, where the anatomical object has grown, shrunk, been surgically altered, or has otherwise experienced a physical change in between the time when the first image data was generated and the time when the second image data was generated, the updating may comprise updating the digital model to reflect any such alteration of and/or change in the anatomical object.

The method 200 also comprises generating navigation guidance based on the updated digital model (step 220). The navigation guidance may be or comprise, or correspond to, a tool trajectory and/or path to be used by or for a surgical tool to carry out a surgical task involving the anatomical element. For example, the tool trajectory and/or path may comprise a trajectory along which to drill a hole in a vertebra in preparation for implantation of a pedicle screw therein. The tool trajectory and/or path may comprise a path along which an incision will be made in soft anatomical tissue, or along which a portion of bony anatomy will be removed. Because any surgical trajectory and/or path may need to be highly accurate to avoid causing damage to surrounding tissue (including, for example, nerves and sensitive anatomical elements) and/or undue trauma to the patient, the ability to generate navigation guidance based on an updated digital model that accurately reflects a real-time or near real-time position of the anatomical object represents a highly beneficial advantage of the present disclosure.

The navigation guidance may be utilized by a robot to control a robotic arm to carry out a surgical task utilizing one or more surgical tools. Alternatively, the navigation guidance may be communicated to a surgeon or other user (e.g., graphically via a screen, or in any other suitable manner), to enable the surgeon or other user to move a surgical tool along a particular trajectory and/or path.

In some embodiments, the method 200 may comprise generating robotic guidance based on the updated digital model. For example, where a robot with an accurate robotic arm is used in connection with a surgical procedure, the method 200 may comprise generating guidance for moving the accurate robotic arm (and, more particularly, for causing the accurate robotic arm to move a surgical tool) along a particular trajectory or path based on a pose of the anatomical object in the updated digital model. In such embodiments, a navigation system may be used to confirm accurate movement of the robotic arm (or more particularly of the surgical tool) along the particular trajectory or path, or a navigation system may not be used in connection with the surgical procedure.

The method 200 also comprises changing a predetermined tool trajectory based on the updated digital model (step 224). Where a tool trajectory is predetermined in a surgical plan or elsewhere based on, for example, the first image data and/or other preoperative information, movement of the anatomical element after the first image data and/or other preoperative information is obtained may necessitate updating of the predetermined tool trajectory so that the tool is maneuvered into the correct pose and along the correct path relative to the anatomical object. Thus, in the step 224, the predetermined tool trajectory may be updated based on the digital model, which reflects the pose of the anatomical object based on the second image data, to yield an updated tool trajectory that maintains a desired positioning of the surgical tool relative to the anatomical object.

As a basic example, if a particular anatomical object were to rotate five degrees from an original position represented in the first image data and a recent position represented in the second image data, and a predetermined tool trajectory had been identified based on the original position of the anatomical object, then the predetermined tool trajectory would also need to be rotated five degrees (about the same axis as the anatomical object) to maintain the predetermined positioning of the surgical tool relative to the anatomical object. In reality, an anatomical object may rotate, translate, experience changes in size and/or relative dimensions (e.g., length vs. height), and/or move or change in one or more other ways in between generation of first image data representing the anatomical object and second image data representing the anatomical object. Regardless of what movements of or changes in the anatomical object occur, the step 224 may be utilized to ensure that a predetermined tool trajectory is properly modified in light of such movements or changes (as reflected in the updated digital model), so that a surgical procedure is carried out with respect to the anatomical object as planned.

The method 200 also comprises measuring an anatomical angle based on the updated digital model (step 228). The anatomical angle may be, for example, an angle created by two surfaces of the anatomical object, or any other angle created by the anatomical object. In some embodiments, the anatomical angle may be an angle between the anatomical object and an adjacent anatomical element, such as a Cobb angle. In some embodiments, the anatomical angle may be an angle between the anatomical object and a reference plane (e.g., a horizontal plane, a vertical plane, or another reference plane). In some embodiments, the anatomical angle may be defined in part by an imaginary line tangent to a surface of the anatomical object, and/or of another anatomical element. The anatomical angle may be an angle that is dependent on a pose of the anatomical object, such that movement of the anatomical object results in a change of the anatomical angle. Embodiments of the present disclosure beneficially enable measurement of such an anatomical angle based on a real-time or near real-time position of the anatomical object (as reflected in the updated digital model).

In some embodiments, the measuring comprises measuring a parameter other than an anatomical angle (whether instead of or in addition to measuring an anatomical angle). The parameter may be, for example, a distance, a radius of curvature, a length, a width, a circumference, a perimeter, a diameter, a depth, or any other useful parameter.

The method 200 also comprises receiving a surgical plan comprising a target anatomical angle (step 232). The surgical plan may be the same as or similar to the surgical plan 134. The target anatomical angle may reflect or otherwise represent a desired outcome of the surgical procedure during which the second image data is generated and received. Thus, for example, if one measure of success of a surgical procedure is whether a particular Cobb angle has been achieved, then the surgical plan may comprise a target Cobb angle. The surgical plan may, however, comprise any target anatomical angle, and the target anatomical angle may or may not reflect a degree of success of the surgical procedure. The target anatomical angle may be an angle that needs to be achieved in order for a subsequent task of the surgical procedure to be completed.

In some embodiments, the surgical plan may comprise a target parameter other than a target anatomical angle (whether instead of or in addition to the target anatomical angle). The target parameter may be, for example, a distance, a radius of curvature, a length, a width, a circumference, a perimeter, a diameter, a depth, or any other parameter of interest.

Also in some embodiments, the step 232 may comprise updating the surgical plan based on the measured anatomical angle (or other measured anatomical parameter) to yield an updated surgical plan. For example, the surgical plan may be updated to include one or more surgical tasks or procedures for achieving the target anatomical angle given the measured anatomical angle or other parameter. The updating may be accomplished automatically (using, for example, historical data regarding appropriate methods of achieving a target anatomical angle, or any other algorithm or data) or based on input received from a surgeon or other user. In some embodiments, the updating may comprise automatically generating one or more recommended surgical tasks or procedures to be added to the surgical plan, and then updating the surgical plan to include one or more of the recommended surgical tasks or procedures based on user input.

The method 200 also comprises comparing the measured anatomical angle to the target anatomical angle (step 236). The comparing may comprise evaluating whether the measured anatomical angle is equal to the target anatomical angle. The comparing may comprise evaluating whether the measured anatomical angle is within a predetermined range of the target anatomical angle (e.g., within one degree, or within two degrees, or within three degrees, or within four degrees, or within five degrees), or by what percentage the measured anatomical angle differs from the target anatomical angle (e.g., by one percent, or by five percent, or by ten percent). In some embodiments, the purpose of the comparing may be to determine whether a given surgical task is complete or, alternatively, needs to be continued. In other embodiments, the purpose of the comparing may be to evaluate a level of success of a surgical task or of the surgical procedure. In still other embodiments, the purpose of the comparing may be to facilitate determination of one or more subsequent surgical tasks. The comparing may utilize one or more algorithms, including any algorithm described herein. In some embodiments, the results of the comparing may be displayed or otherwise presented to a user via a user interface such as the user interface 110. Also in some embodiments, the results of the comparing may trigger an alert or a warning if those results exceed or do not reach a predetermined threshold.

In embodiments of the method 200 in which a parameter other than an anatomical angle is measured, and the surgical plan comprises a target parameter other than a target anatomical angle, the comparing may comprise comparing the measured parameter to the target parameter.

The method 200 also comprises displaying the updated digital model on a user interface (step 240). The user interface may be the same as or similar to the user interface 110. The updated digital model may be displayed, for example, on a user interface comprising a screen. In some embodiments, the updated digital model may be displayed on a touchscreen that enables manipulation of the model (e.g., zooming in, zooming out, rotating, translating). Also in some embodiments, a surgeon or other user may be able to manipulate one or more aspects of a surgical plan (such as, for example, the surgical plan 134) using the displayed digital model.

The present disclosure encompasses embodiments of the method 200 that comprise more or fewer steps than those described above, and/or one or more steps that are different than the steps described above.

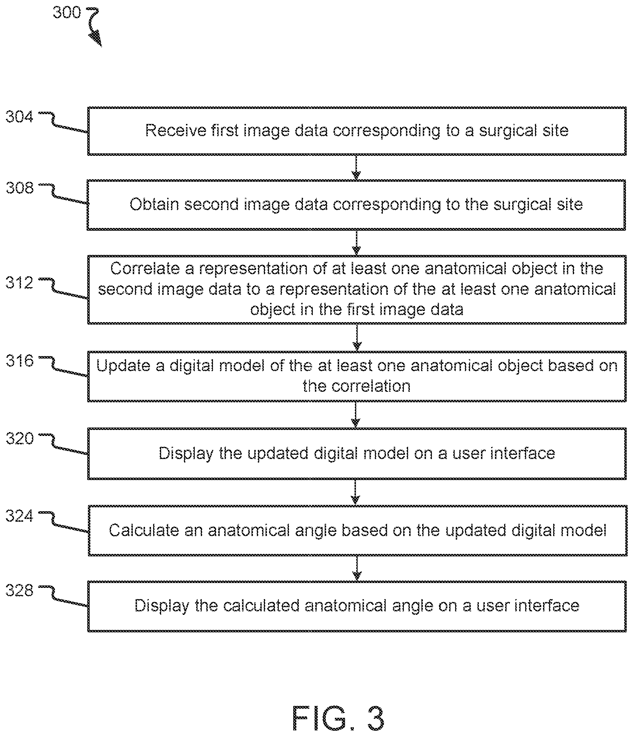

Turning now to Fig. 3, embodiments of the present disclosure may be used, for example, for segmental tracking during a surgical procedure. The surgical procedure may be a spinal surgery or any other surgical procedure.

Fig. 3 depicts a segmental tracking method 300. The segmental tracking method 300 (and/or one or more steps thereof) may be carried out or otherwise performed, for example, by at least one processor. The at least one processor may be the same as or similar to the processor(s) 104 of the computing device 102 described above. The at least one processor may be part of a robot (such as a robot 130) or part of a navigation system (such as a navigation system 114). A processor other than any processor described herein may also be used to execute the method 300. The at least one processor may perform the method 300 by executing instructions stored in a memory such as the memory 106. The instructions may correspond to one or more steps of the method 300 described below. The instructions may cause the processor to execute one or more algorithms, such as the image processing algorithm 120, the feature recognition algorithm 122, the segmentation algorithm 124, the fiducial detection algorithm 126, and/or the model update or comparison algorithm 128.

The method 300 comprises receiving first image data corresponding to a surgical site of a patient (step 304). The step 304 may be the same as or substantially similar to the step 204 of the method 200 described above.

The method 300 also comprises obtaining second image data corresponding to the surgical site (step 308). The step 308 may be the same as or substantially similar to the step 208 of the method 200 described above. The obtaining may comprise causing an imaging device to obtain an image of the surgical site. The imaging device may be, for example, an ultrasound probe or a radar system. In some embodiments, the imaging device may be the same imaging device used to obtain the first image data. Where the imaging device is an ultrasound probe, the device may be or comprise an ultrasound transducer and an ultrasound receiver in a common housing, or a transducer and a receiver that are physically separate. Alternatively, the imaging device may be a radar system comprising, for example, a transmitter (including a transmitting antenna) and receiver (including a receiving antenna). The transmitter and the receiver may be contained within a common housing or may be provided in physically separate housings.

The second image data may be or comprise topographic image data, tomographic image data, or another kind of image data. The second image data is obtained during a surgical procedure in real-time (e.g., with a delay of 500 milliseconds or less, or a delay of 250 milliseconds or less) or in near real-time (such that, e.g., less than one minute, less than thirty seconds, less than ten seconds, or less than five seconds, or less than one second passes from the moment the second image data is generated by the imaging device to the moment the second image data is obtained from the imaging device). The second image data is obtained after the first image data is received.

The method 300 also comprises correlating a representation of at least one anatomical object in the second image data to a representation of the at least one anatomical object in the first image data (step 312). The step 312 may be the same as or similar to the step 212 of the method 200 described above. The correlating may occur for just one anatomical element, or for more than one anatomical element. For example, where the surgical site comprises a portion of a patient's spine, the correlating may occur for each of a plurality of vertebrae included within the portion of the patient's spine.

The method 300 also comprises updating a digital model of the at least one anatomical object based on the correlation (step 316). The step 316 may be the same as or similar to the step 216 of the method 200 described above.

The method 300 also comprises displaying the updated digital model on a user interface (step 320). The step 320 may be the same as or similar to the step 240 of the method 200 described above.

The method 300 also comprises calculating an anatomical angle based on the updated digital model (step 324). In some embodiments, the calculating may comprise measuring one or more parameters on the updated digital model, and using the measured one or more parameters to calculate the anatomical angle. The anatomical angle may be the same as or similar to any other anatomical angle described herein. In other embodiments, the calculating may simply comprise measuring the anatomical angle. The step 324 may, in some embodiments, be the same as or similar to the step 228.

Although the step 324 is described with respect to calculating an anatomical angle, in other embodiments the step 324 may additionally or alternatively comprise calculating and/or measuring a parameter other than an anatomical angle. The parameter may be any anatomical parameter described herein, including, for example, any parameter described above in connection with the steps 228, 232, and/or 236 of the method 200.

The method 300 also comprises displaying the calculated anatomical angle on a user interface (step 328). The calculated anatomical angle may be displayed, for example, on a screen of a user interface such as a user interface 110. The angle may be displayed as a stand-alone number, or may be superimposed on (or otherwise displayed in connection with) the digital model. In some embodiments, the angle may be displayed as a number and the lines or surfaces forming the angle may be highlighted or otherwise identified in a displayed digital model. In some embodiments, a surgeon or other user may be able to select a pair of surfaces, a pair of lines, a surface and a line, or any other objects in the digital model forming an angle, and the angle between the selected objects may then be calculated (as described above, for example, in connection with the step 324) and displayed on the user interface.

The present disclosure encompasses embodiments of the method 300 that comprise more or fewer steps than those described above, and/or one or more steps that are different than the steps described above.

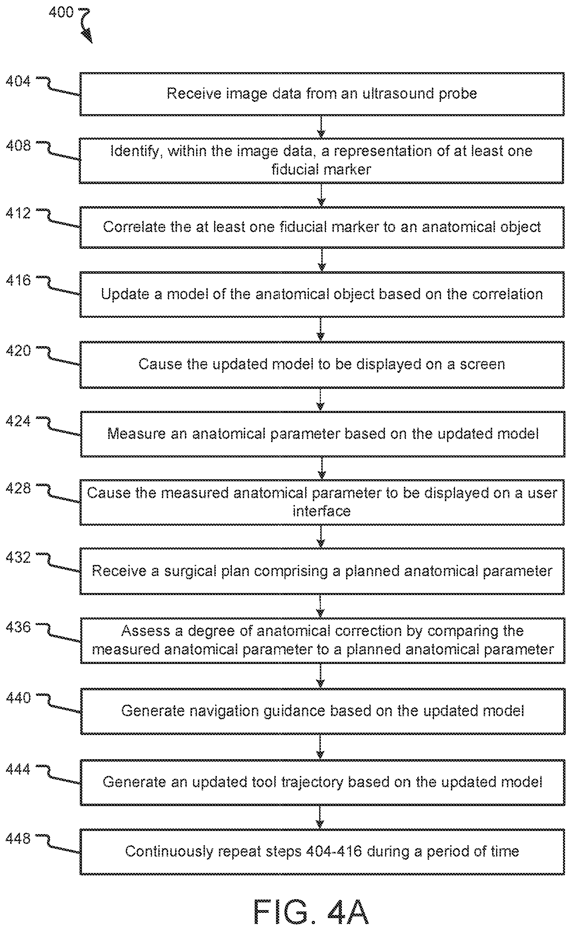

Fig. 4A depicts a method 400 for segmental tracking. The segmental tracking method 400 (and/or one or more steps thereof) may be carried out or otherwise performed, for example, by at least one processor. The at least one processor may be the same as or similar to the processor(s) 104 of the computing device 102 described above. The at least one processor may be part of a robot (such as a robot 130) or part of a navigation system (such as a navigation system 114). A processor other than any processor described herein may also be used to execute the method 400. The at least one processor may perform the method 400 by executing instructions stored in a memory such as the memory 106. The instructions may correspond to one or more steps of the method 400 described below. The instructions may cause the processor to execute one or more algorithms, such as the image processing algorithm 120, the feature recognition algorithm 122, the segmentation algorithm 124, the fiducial detection algorithm 126, and/or the model update or comparison algorithm 128.

The method 400 comprises receiving image data from an ultrasound probe (step 404). The ultrasound probe may be a single ultrasound probe or a plurality of ultrasound probes. The image data may be or comprise topographic data and/or tomographic data. The image data may be received in real-time from the ultrasound probe (e.g., within 500 milliseconds or within 250 milliseconds of the image data being generated by the ultrasound probe), or the image data may be received in near real-time from the ultrasound probe (e.g., within one minute, or within thirty seconds, or within ten seconds, or within five seconds, or within one second from the time the image data is generated by the ultrasound probe). The ultrasound probe may be or comprise a transducer and a receiver in a common physical housing, immediately adjacent to each other, or otherwise having a fixed position relative to each other, or the ultrasound probe may be or comprise a transducer and a receiver that are physically separate and independently movable. In some embodiments, the receiver may be configured to receive ultrasonic signals that are emitted by the transducer and reflected by one or more anatomical elements within a field of view of the imaging device. In other embodiments, the ultrasound probe may be only an ultrasound receiver, which may be configured to receive ultrasonic signals generated by an active fiducial marker.

The image data may represent or otherwise correspond to a surgical site or an intended surgical site of a patient. For example, if a patient is to undergo spinal surgery, then the image data may represent or otherwise correspond to the portion of the patient's spine and surrounding anatomy at which the surgical procedure will take place. As another example, if the patient is to undergo knee surgery, then the image data may represent or otherwise correspond to the patient's knee or a portion thereof.

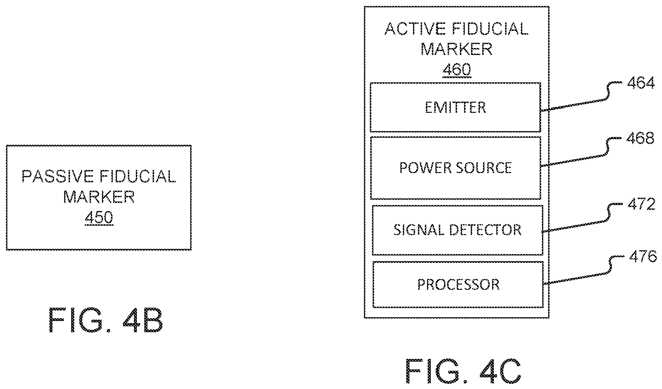

The method 400 also comprises identifying, within the image data, a representation of at least one fiducial marker (step 408). The fiducial marker may be, for example, a fiducial marker optimized to be detected by an ultrasound transducer. The fiducial marker may be passive or active. Passive fiducial markers, such as the fiducial marker 450 shown in Fig. 4B, may be acoustically reflective (e.g., echogenic) and of a particular identifiable shape that could be localized in an ultrasound image. Different passive fiducial markers may be configured to have unique echogenic properties relative to one or more other passive fiducial markers, such that individual passive fiducial markers can be specifically identified and distinguished from other passive fiducial markers represented in the image data. The fiducial markers may be metallic, polymeric, or a combination thereof. The fiducial markers may or may not be bioabsorbable.

Active fiducial markers, such as the fiducial marker 460 shown in Fig. 4C, may comprise an emitter 464 configured to generate ultrasonic or other sound waves (or other emissions tailored for the imaging modality with which the fiducial markers are to be used). In some embodiments, such active fiducial markers may comprise a power source 468 for powering the emitter 464 and other components of the fiducial marker 460; a signal detector 472; and a processor 476 or other circuitry that enables the emitter 464 to emit noise at a particular frequency, whether constantly, or at predetermined intervals, or in response to a signal detected by the signal detector 472 (whether an ultrasonic signal or otherwise). In some embodiments, an active fiducial marker according to some embodiments of the present disclosure may comprise a piezo-electric speaker as the emitter, connected to a power source that is either co-located with the piezo-electric speaker (e.g., within a common housing) or located remote from the piezo-electric speaker but connected thereto with a wire.

In other embodiments, such active fiducial markers may not comprise a power source, but instead may be configured to vibrate when within an electromagnetic field. Active fiducial markers that are activated by an electromagnetic field may beneficially have a smaller size and/or be placed in more difficult-to-access locations than active fiducial markers that require a physically connected power source (whether that power source is within a housing of the fiducial marker or connected thereto with a wire or cable). Any active fiducial marker described herein may be configured to emit ultrasonic or other sound waves at a particular frequency, which frequency may or may not be selectable by a user of the fiducial marker (e.g., prior to or after attachment of the fiducial marker to an anatomical element). Fiducial markers configured to emit ultrasonic or other sound waves at unique frequencies relative to each other may be attached to one or more anatomical elements of a patient, to facilitate identification of each fiducial marker (and its corresponding anatomical element) in an ultrasound image.

Whether passive or active fiducial markers are used, at least one fiducial marker may be placed on each anatomical element of interest. For example, where fiducial markers are being used in connection with a spinal procedure, at least one fiducial marker may be placed on each vertebra in a region of interest of a patient's spine. The fiducial markers may beneficially be used to facilitate registration of an image space to a patient space, and/or registration of a first image space to a second image space or vice versa. In other words, the fiducial markers may be used to match corresponding anatomical elements in an image space and a patient space, or in a first image space and a second image space, so that a spatial relationship between the two spaces in question may be determined. In some embodiments, the use of a single fiducial marker on each vertebra (or other anatomical element) may be sufficient to determine a pose (position and orientation) of the vertebra. In other embodiments, a plurality of fiducial markers may be placed on each vertebra to facilitate determination of the pose of the vertebra.

Also, in some embodiments of the present disclosure the method 400 may comprise a step of triangulating or otherwise locating a position (and/or determining an orientation) of the at least one fiducial marker other than by identifying a representation of the at least one fiducial marker in image data. In such embodiments, the triangulating or otherwise locating the fiducial marker may utilize a plurality of sensors (which may or may not include one or more imaging devices) to detect a signal generated or reflected by the fiducial marker. A fiducial marker detection algorithm such as the algorithm 126 may then be used to calculate a position of the fiducial marker (and/or to determine an orientation of the fiducial marker) based on information corresponding to the detected signals.

The method 400 also comprises correlating the at least one fiducial marker to an anatomical object (step 412). Where the at least one fiducial marker comprises a plurality of indistinguishable fiducial markers, the correlating may utilize the fiducial markers to facilitate detection of individual anatomical segments in the image. In other words, the fiducial markers may be used in conjunction with one or more segmentation algorithms and/or other algorithms to identify, within the image, individual anatomical elements and then to associate each fiducial marker to one of the individual anatomical elements. Where the at least one fiducial marker comprises a plurality of distinguishable fiducial markers, the correlating may comprise, in some embodiments, the same steps as described above for a plurality of indistinguishable fiducial markers. In other embodiments, the correlating may comprise accessing a database or lookup table to obtain information about the anatomical element to which each fiducial marker is attached, and utilizing that information to facilitate identification of the anatomical element in the image data and correlation of the corresponding fiducial marker therewith.

Independent of how the correlating occurs, the step 412 enables determination, based on information about the at least one fiducial marker in the image data, of a pose of the anatomical element to which the at least one fiducial marker is attached. Where the at least one fiducial marker comprises a plurality of fiducial markers, the step 412 enables determination of a pose of each of the anatomical elements to which each of the plurality of fiducial markers are attached.

The method 400 also comprises updating a model of the anatomical object based on the correlation (step 416). The model of the anatomical object may be a 2D image or a 3D image. The model may be generated by an imaging device such as the imaging device 112, or by compiling or otherwise combining a plurality of images from an imaging device such as the imaging device 112. The model may, in some embodiments, be generated using a CAD program or other visualization and/or modeling software. The model is segmented, such that individual elements within the model may be moved relative to other individual elements within the model. Thus, where the model is a model of a spine or a portion thereof, and the anatomical object is a vertebra, at least the vertebra that is the anatomical object is movable relative to the other vertebrae of the spine or portion thereof. In some embodiments, all of the vertebrae in the spine or portion thereof are movable relative to each other. As a result, the model may be updated to reflect the most recently identified pose of the anatomical object. Where the method 400 is carried out in real-time or in near real-time, the model may be updated to reflect the current pose (or nearly current pose) of the anatomical object.

The updating may comprise determining a pose of the anatomical object relative to a predetermined coordinate system (e.g., a navigation coordinate system, a patient coordinate system, a robotic coordinate system). Where the model is already registered to the predetermined coordinate system, the updating may comprise adjusting the pose of the modeled anatomical object to match the pose of the real-life anatomical object. Where the model is not already registered to the predetermined coordinate system, a registration process may be completed prior to the model being updating. Alternatively, the pose of the anatomical object may be determined relative to a pose of an adjacent anatomical element, and the model may be updated to reflect the same relative pose between the anatomical object and the adjacent anatomical element. In still other embodiments, any anatomical element, implant, or other suitable reference point that is present in both the image data and the model may be selected as a reference for determining a pose of the anatomical object, and the updating may comprise modifying a pose of the modeled anatomical object relative to the reference to match a determined pose of the actual anatomical object relative to the reference.

The method 400 also comprises causing the updated model to be displayed on a screen (step 420). The screen may be any screen visible by a surgeon or other person participating in and/or monitoring the patient and/or the surgical procedure. The screen may be a user interface 110, or a part of a navigation system such as the navigation system 114, or any other screen. The screen may be a touchscreen, and the displayed model may be manipulatable by a surgeon or other user. For example, the surgeon or other user may be able to zoom in or out, rotate the model, pan the model around the screen, and/or otherwise adjust a view of the model. Also in some embodiments, the surgeon or other user may be able to view a surgical plan overlaid on the model, where the surgical plan comprises, for example, one or more proposed implants (e.g., screws, rods, interbodies) and/or insertion trajectories for such implants.

The method 400 also comprises measuring an anatomical parameter based on the updated model (step 424). The anatomical parameter may be, for example, a length, width, diameter, radius of curvature, circumference, perimeter, and/or depth of the anatomical object or any other anatomical element; a distance between the anatomical object and another object (e.g., another anatomical element, an implant); a distance between an implant and anatomical surface; a distance and/or angle between the anatomical object or a portion thereof and a reference line or plane; an angle formed by the anatomical object with one or more other anatomical elements and/or implants; a parameter descriptive of a position of an implant relative to the anatomical object or another anatomical element; a Cobb angle; an angle formed between two lines and/or surfaces defined, in whole or in part, by the anatomical object and/or another anatomical element; or any other parameter of interest. The measurement may be obtained solely from the updated model, or the measurement may be obtained by overlaying a reference line or plane, a surgical plan, and/or other information on the updated model.

The method 400 also comprises causing the measured anatomical parameter to be displayed on a user interface (step 428). The user interface may be the screen on which the updated model is displayed in the step 420. The user interface may alternatively be any other user interface, including a user interface that is the same as or similar to the user interface 110. The parameter may be displayed as a stand-alone number, or may be superimposed on (or otherwise displayed in connection with) the updated model of the anatomical object. In some embodiments, the parameter may be displayed as a number, and the lines or surfaces from which the parameter was measured may be highlighted or otherwise identified in the displayed model. In some embodiments, a surgeon or other user may be able to select, using a screen or other user interface as well as the displayed model, an anatomical parameter to be measured using the model. The anatomical parameter may then be measured and displayed on the user interface.

The method 400 also comprises receiving a surgical plan comprising a planned anatomical parameter. The surgical plan may be the same as or similar to the surgical plan 134 and may comprise one or more planned or target anatomical parameters to be achieved during a surgical procedure described in the surgical plan. The one or more planned anatomical parameters may be or include, for example, a target implant depth, a target distance between an implant and an anatomical surface (e.g., a pedicle wall); a target diameter of a hole to be drilled in the anatomical object or other anatomical element; a target angle to be achieved between the anatomical object and one or more other anatomical elements through the use of one or more implants; a target radius of curvature to be created by removing a portion of bony anatomy; and/or any other target anatomical parameters. The surgical plan may comprise one or more models of implants, which models may be superimposed over or otherwise displayed together with the model of the anatomical object. The surgical plan may also comprise one or more implant insertion trajectories, tool trajectories, tool models, tool specifications, and/or other information useful for a surgeon or other medical attendant for defining and/or properly carrying out a surgical procedure. Any of the foregoing information may be displayed on a screen, whether together with or separate from the model of the anatomical object.

The method 400 may also comprise assessing a degree of anatomical correction by comparing the measured anatomical parameter (of the step 424) to the planned anatomical parameter (of the step 432) (step 436). The comparing may comprise evaluating whether the measured anatomical parameter is equal to the planned or target anatomical parameter. The comparing may comprise evaluating whether the measured anatomical parameter is within a predetermined range of the planned anatomical parameter (e.g., within one degree, or within two degrees, or within three degrees, or within four degrees, or within five degrees), or by what percentage the measured anatomical parameter differs from the planned anatomical parameter (e.g., by one percent, or by five percent, or by ten percent). In some embodiments, the purpose of the comparing may be to determine whether a given surgical task is complete or, alternatively, needs to be continued. In other embodiments, the purpose of the comparing may be to evaluate a level of success of a surgical task or of the surgical procedure. In still other embodiments, the purpose of the comparing may be to facilitate determination of one or more subsequent surgical tasks. The comparing may utilize one or more algorithms. In some embodiments, the results of the comparing may be displayed or otherwise presented to a user via a user interface such as the user interface 110. Also in some embodiments, the results of the comparing may trigger an alert or a warning if those results exceed or do not reach a predetermined threshold.

The method 400 also comprises generating navigation guidance based on the updated model (step 440). The navigation guidance may be or comprise, or correspond to, a tool trajectory and/or path to be used by or for a surgical tool to carry out a surgical task involving the anatomical object. For example, the tool trajectory and/or path may comprise a trajectory along which to drill a hole in a vertebra in preparation for implantation of a pedicle screw therein. The tool trajectory and/or path may comprise a path along which an incision will be made in soft anatomical tissue, or along which a portion of bony anatomy will be removed. Because any surgical trajectory and/or path may need to be highly accurate to avoid causing damage to surrounding tissue and/or undue trauma to the patient, the ability to generate navigation guidance based on the updated model, which accurately reflects a real-time or near real-time position of the anatomical object, represents a highly beneficial advantage of the present disclosure.

The navigation guidance may be utilized by a robot to control a robotic arm to carry out a surgical task utilizing one or more surgical tools. Alternatively, the navigation guidance may be communicated to a surgeon or other user (e.g., graphically via a screen, or in any other suitable manner), to enable the surgeon or other user to move a surgical tool along a particular trajectory and/or path.

In some embodiments, the method 400 may comprise generating robotic guidance based on the updated model (e.g., from the step 416). For example, where a robot with an accurate robotic arm is used in connection with a surgical procedure, the method 400 may comprise generating guidance for moving the accurate robotic arm (and, more particularly, for causing the accurate robotic arm to move a surgical tool) along a particular trajectory or path based on a pose of the anatomical object in the updated model. In such embodiments, a navigation system may be used to confirm accurate movement of the robotic arm (or more particularly of the surgical tool) along the particular trajectory or path, or a navigation system may not be used in connection with the surgical procedure.

The method 400 also comprises generating an updated tool trajectory based on the updated model (step 444). Where a tool trajectory is predetermined in a surgical plan or elsewhere based on, for example, a preoperative image and/or other preoperative information, movement of the anatomical element after the preoperative image and/or other preoperative information is obtained may necessitate updating of the predetermined tool trajectory so that the tool is maneuvered into the correct pose and along the correct path relative to the anatomical object. Thus, in the step 444, the predetermined tool trajectory may be updated based on the updated model, which reflects the pose of the anatomical object based on the image data from the ultrasound probe, to yield an updated tool trajectory that maintains a desired positioning of the surgical tool relative to the anatomical object. The generating may be the same as or similar to the changing a predetermined tool trajectory based on an updated digital model, described above in connection with the step 224 of the method 200.

The method 400 also comprises continuously repeating at least the steps 404 through 416 during a period of time (step 448). In embodiments of the present disclosure, an ultrasound probe may be operated continuously during some or all of a surgical procedure. The ultrasound probe may be maintained in a fixed position during the surgical procedure, or in a fixed position relative to the patient, or the ultrasound probe may be positioned on a movable robotic arm or another movable support, and may be moved from one pose to another during the course of the surgical procedure. Independent of its pose, the ultrasound probe may generate a stream of image data, which stream of image data may contain a representation of each of the at least one fiducial markers. As the stream of image data is received, the representation(s) of the at least one fiducial markers may be identified and correlated to a corresponding anatomical object, based upon which correlation the model of the anatomical object may be updated. Thus, the updating may occur continuously (e.g., in real-time or in near real-time) or at predetermined intervals (which intervals may be separated by less than thirty seconds, or by less than one minute, or by less than five minutes). As a result, the model of the at least one anatomical object may be continuously and/or repeatedly updated during a surgical procedure to reflect the position of each of the at least one anatomical objects, thus facilitating the accurate completion of the surgical procedure.

In addition to repeating the steps 404 through 416 during a period of time, one or more of the steps 420 through 444 may also be repeated (whether continuously or not) during the period of time.

Although the method 400 is described herein as utilizing image data from an ultrasound probe and ultrasonic fiducial markers, other embodiments of the method 400 may utilize other imaging modalities and fiducial markers that correspond thereto. Thus, for example, in an embodiment of the method 400 according to the invention, the step 404 comprises receiving image data from a radar system, and the step 408 comprises identifying within the image data a representation of at least one radar-specific fiducial marker.

The present disclosure encompasses embodiments of the method 400 that comprise more or fewer steps than those described above, and/or one or more steps that are different than the steps described above.

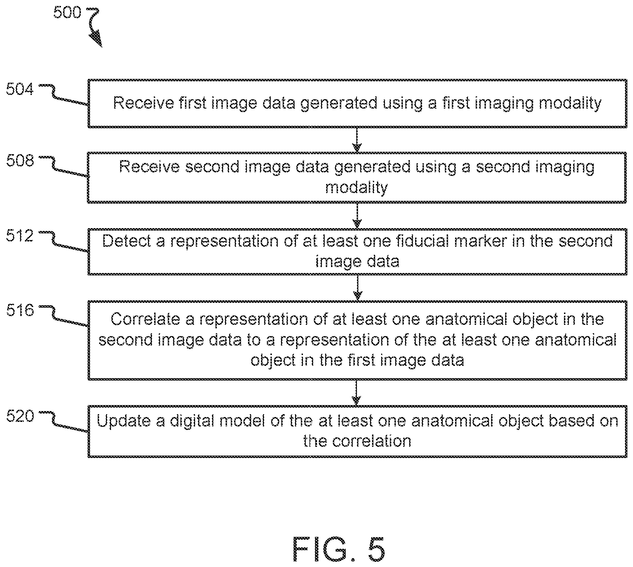

Fig. 5 depicts a method 500 for segmental tracking. The segmental tracking method 500 (and/or one or more steps thereof) may be carried out or otherwise performed, for example, by at least one processor. The at least one processor may be the same as or similar to the processor(s) 104 of the computing device 102 described above. The at least one processor may be part of a robot (such as a robot 130) or part of a navigation system (such as a navigation system 114). A processor other than any processor described herein may also be used to execute the method 500. The at least one processor may perform the method 500 by executing instructions stored in a memory such as the memory 106. The instructions may correspond to one or more steps of the method 500 described below. The instructions may cause the processor to execute one or more algorithms, such as the image processing algorithm 120, the feature recognition algorithm 122, the segmentation algorithm 124, the fiducial detection algorithm 126, and/or the model update or comparison algorithm 128.

The method 500 comprises receiving first image data generating using a first imaging modality (step 504). The step 504 may be the same as or similar to the step 204 of the method 200 described above. The first image data may be or correspond to, for example, a preoperative image, whether received as a standalone image or as part of a surgical plan.

The method 500 also comprises receiving second image data generated using a second imaging modality (step 508). The step 508 may be the same as or similar to the step 208 of the method 200 described above. In particular, the second imaging modality may be, for example, ultrasound or radar. In embodiments according to the invention, the second imaging modality utilizes radar.

Either or both of the first image data and the second image data may be or comprise topographic data and/or tomographic data.

The method 500 also comprises detecting a representation of at least one fiducial marker in the second image data (step 512). Where the second imaging modality of the step 508 is ultrasound, the step 512 may be the same as the step 408 of the method 400 described above. Where the second imaging modality of the step 508 is radar, the step 512 may be similar to the step 408 of the method 400 described above, but with active or passive fiducial markers intended for use with radar imagers. In embodiments according to the invention, an active fiducial marker is configured to generate electromagnetic waves having an appropriate frequency for detection by the particular radar imager being used, while passive fiducial markers, which do not form part of the invention, may have a structure optimized to reflect electromagnetic waves generated by the radar imager being used. In some embodiments, the fiducial markers may be transponders, in that they are configured to transmit an electromagnetic wave at a particular frequency in response to receiving and/or detecting an electromagnetic wave from the radar imager at a different frequency.

Where the second imaging modality utilizes an imaging technology other than ultrasound or radar, the at least one fiducial marker of the step 512 may be selected to be compatible with the utilized imaging technology.

The method 500 also comprises correlating a representation of at least one anatomical object in the second image data to a representation of the at least one anatomical object in the first image data (step 516). The step 516 may be the same as or similar to the step 212 of the method 200 described above. However, the step 516 may also comprise correlating the representation of the at least one fiducial marker detected in the step 512 with at least one anatomical object in the second image data. Thus, for example, where the at least one fiducial marker comprises a plurality of indistinguishable fiducial markers, the correlating may utilize the fiducial markers to facilitate detection of individual anatomical segments, elements, or objects in the image. In other words, the fiducial markers may be used in conjunction with one or more segmentation algorithms and/or other algorithms to identify, within the image, individual anatomical elements and then to associate each fiducial marker to one of the individual anatomical elements. As another example, where the at least one fiducial marker comprises a plurality of distinguishable fiducial markers, the correlating may comprise, in some embodiments, the same steps as described above for a plurality of indistinguishable fiducial markers, while in other embodiments the correlating may comprise accessing a database or lookup table to obtain information about the anatomical element to which each fiducial marker is attached, and utilizing that information to identify the anatomical element in the image data and correlate the corresponding fiducial marker therewith.

In embodiments where the representation of the at least one fiducial marker in the second image data is correlated to a corresponding anatomical object or objects in the second image data, the results of that correlating may be used in connection with the correlating the representation of the at least one anatomical object in the second image data to a representation of the at least one anatomical object in the first image data. In other words, the fiducial markers may be used to help identify the representation of the at least one anatomical object in the second image data, which may then be correlated to the representation of the at least one anatomical object in the first image data. In embodiments where a database or lookup table comprises information about the anatomical object to which each fiducial marker is attached, the information in the database or lookup table may be used to facilitate correlating the representation of the at least one anatomical object in the second image data to a corresponding representation of the at least one anatomical object in the first image data.

The method 500 also comprises updating a digital model of the at least one anatomical object based on the correlation (step 520). The step 520 may be the same as or similar to the step 216 of the method 200 described above. The updating may also be based on, for example, the detected representation of the at least one fiducial marker in the second image data, which may facilitate determination of a pose of the at least one anatomical object in the second image data, and thus facilitate updating the digital model to reflect the pose of the at least one anatomical object as reflected in the second image data. The result of the updating may be, for example, a digital model in which the position of the at least one anatomical object has been updated to match a pose of the at least one anatomical object in the second image data.

As with other methods described herein, the method 500 beneficially enables a digital model of a surgical site or other anatomical portion of a patient to be updated, either in real-time or in near real-time, to reflect changes in the position of one or more anatomical objects represented thereby. Ensuring that the digital model reflects a real-time or near real-time position of the one or more anatomical elements beneficially enables, for example: a surgeon to plan a subsequent step of a surgical procedure based on an actual position of relevant anatomical objects, rather than using preoperative or other images that no longer accurately represent the surgical site or other anatomical portion of the patient; a navigation system to generate accurate navigation guidance, whether for a surgeon or a robot, based on the actual position of the one or more relevant anatomical elements; and/or a robotic system to execute a surgical procedure with the correct trajectory relative to the actual pose of the one or more affected anatomical elements.

The present disclosure encompasses embodiments of the method 500 that comprise more or fewer steps than those described above, and/or one or more steps that are different than the steps described above.

As noted above, the present disclosure encompasses methods with fewer than all of the steps identified in Figs. 2, 3, 4A, and 5 (and the corresponding description of the methods 200, 300, 400, and 500), as well as methods that include additional steps beyond those identified in Figs. 2, 3, 4A, and 5 (and the corresponding description of the methods 200, 300, 400, and 500). The present disclosure also encompasses methods that comprise one or more steps from one method described herein, and one or more steps from another method described herein. Any correlation described herein may be or comprise a registration or any other correlation.

Aspects of the present disclosure may be utilized in conjunction with one or more aspects of U.S. Patent Application Serial No. 63/052,763, filed July 16, 2020 and entitled "System and Method for Image Generation Based on Calculated Robotic Arm Positions"; U.S. Patent Application Serial No. 63/052,766, filed July 16, 2020 and entitled "System and Method for Image Generation Based on Calculated Robotic Arm Positions"; and/or U.S. Patent Application No. 16/984,514, filed August 4, 2020 and entitled "Triangulation of Item in Patient Body,".

The foregoing is not intended to limit the disclosure to the form or forms disclosed herein. In the foregoing Detailed Description, for example, various features of the disclosure are grouped together in one or more aspects, embodiments, and/or configurations for the purpose of streamlining the disclosure. The features of the aspects, embodiments, and/or configurations of the disclosure may be combined in alternate aspects, embodiments, and/or configurations other than those discussed above. This method of disclosure is not to be interpreted as reflecting an intention that the claims require more features than are expressly recited in each claim. Rather, as the following claims reflect, inventive aspects lie in less than all features of a single foregoing disclosed aspect, embodiment, and/or configuration. Thus, the following claims are hereby incorporated into this Detailed Description, with each claim standing on its own as a separate preferred embodiment of the disclosure.

Moreover, though the foregoing has included description of one or more aspects, embodiments, and/or configurations and certain variations and modifications, other variations, combinations, and modifications are within the scope of the disclosure, e.g., as may be within the skill and knowledge of those in the art, after understanding the present disclosure. It is intended to obtain rights which include alternative aspects, embodiments, and/or configurations to the extent permitted, including alternate, interchangeable and/or equivalent structures, functions, ranges or steps to those claimed, whether or not such alternate, interchangeable and/or equivalent structures, functions, ranges or steps are disclosed herein, and without intending to publicly dedicate any patentable subject matter.

## Claims

1. A segmental tracking method (400), comprising:
receiving first image data corresponding to a surgical site comprising at least one anatomical object, the first image data generated using a first imaging modality;
receiving second image data (404) corresponding to the surgical site, the second image data generated using a second imaging modality different than the first imaging modality;
detecting (408), in the second image data, a representation of at least one fiducial marker (460);
correlating (412), based at least in part on the detected fiducial marker, a representation of the at least one anatomical object in the second image data to a representation of the at least one anatomical object in the first image data; and
updating (416) a digital model of the at least one anatomical object based on the correlation,
**characterised in that**
the second imaging modality utilizes radar;
the at least one fiducial marker is an at least one active fiducial marker; and
wherein the at least one active fiducial marker is configured to generate electromagnetic waves having an appropriate frequency for detection by a particular radar imager.

2. The segmental tracking method of claim 1, further comprising generating (440) navigation guidance based on the updated digital model.

3. The segmental tracking method of claim 1, further comprising changing (444) a predetermined tool trajectory based on the updated digital model.

4. The segmental tracking method of claim 1, further comprising measuring (424) an anatomical parameter based on the updated digital model.

5. The segmental tracking method of claim 4, further comprising comparing the measured anatomical parameter to a target anatomical parameter,
optionally, further comprising receiving (432) a surgical plan comprising the target anatomical parameter,
further optionally, further comprising updating the surgical plan based on the measured anatomical parameter to yield an updated surgical plan.

6. The segmental tracking method of claim 5, wherein the updated surgical plan comprises at least one surgical task for achieving the target anatomical parameter given the measured anatomical parameter.

7. The segmental tracking method of claim 1, wherein the second image data comprises a data stream, and the correlating and the updating occur in real-time or near real-time.

8. The segmental tracking method of claim 1, further comprising displaying (420) the updated digital model on a user interface (110).

9. The segmental tracking method of claim 1, wherein the second image data comprises topographic data or tomographic data.

10. A segmental tracking system (100) comprising:
at least one fiducial marker (460);
a communication interface (108);
an imaging device (112);
at least one processor (104); and
a memory (106) storing instructions for execution by the at least one processor (104) that, when executed, cause the at least one processor (104) to:
receive, via the communication interface (108), first image data corresponding to a surgical site comprising at least one anatomical object;
obtain, using the imaging device (112), second image data corresponding to the surgical site;
detect, in the second image data, a representation of the at least one fiducial marker (460),
correlate, based at least in part on the detected fiducial marker (460), a representation of the at least one anatomical object in the second image data to a representation of the at least one anatomical object in the first image data; and
update a digital model of the at least one anatomical object based on the correlation,
**characterised in that** the at least one fiducial marker (460) is an at least one active fiducial marker (460), wherein the at least one active fiducial marker (460) is configured to generate electromagnetic waves having an appropriate frequency for detection by a particular radar imager, and wherein the imaging device (112) is configured to generate image data using radar.

11. The segmental tracking system (100) of claim 10, wherein the second image data comprises a data stream, the correlating occurs continuously during receipt of the second image data, and the updating occurs continuously during the correlating.

12. The segmental tracking system (100) of claim 10, further comprising a user interface (110), and wherein the memory (106) stores additional instructions for execution by the at least one processor (104) that, when executed further cause the at least one processor (104) to:
display the updated digital model on the user interface (110).

13. The segmental tracking system (100) of claim 10, wherein the memory (106) stores additional instructions for execution by the at least one processor (104) that, when executed, further cause the at least one processor (104) to:
calculate an anatomical angle based on the updated digital model; and
display the calculated anatomical angle on a user interface (110).

14. The segmental tracking system (100) of claim 10, wherein the at least one anatomical object comprises a plurality of vertebrae.

15. The segmental tracking system (100) of claim 10, wherein the second image data comprises topographic data or tomographic data.

## Patentansprüche

1. Verfahren (400) für segmentales Verfolgen, umfassend:
Empfangen erster Bilddaten, die einer Operationsstelle entsprechen, umfassend mindestens ein anatomisches Objekt, wobei die ersten Bilddaten unter Verwendung einer ersten Bildgebungsmodalität erzeugt werden;
Empfangen zweiter Bilddaten (404), die der Operationsstelle entsprechen, wobei die zweiten Bilddaten unter Verwendung einer zweiten Bildgebungsmodalität, die sich von der ersten Bildgebungsmodalität unterscheidet, erzeugt werden;
Erfassen (408), in den zweiten Bilddaten, einer Darstellung mindestens einer Bezugsmarkierung (460);
Korrelieren (412), basierend mindestens teilweise auf der erfassten Bezugsmarkierung, einer Darstellung des mindestens einen anatomischen Objekts in den zweiten Bilddaten mit einer Darstellung des mindestens einen anatomischen Objekts in den ersten Bilddaten; und
Aktualisieren (416) eines digitalen Modells des mindestens einen anatomischen Objekts basierend auf der Korrelation,
**dadurch gekennzeichnet, dass** die zweite Bildgebungsmodalität Radar nutzt;
die mindestens eine Bezugsmarkierung eine mindestens eine aktive Bezugsmarkierung ist; und
wobei die mindestens eine aktive Bezugsmarkierung konfiguriert ist, um elektromagnetische Wellen, die eine geeigneten Frequenz für Erfassung durch einen speziellen Radar-Bildgeber aufweisen, zu erzeugen.

2. Verfahren für segmentales Verfolgen nach Anspruch 1, ferner umfassend das Erzeugen (440) einer Navigationsführung basierend auf dem aktualisierten digitalen Modell.

3. Verfahren für segmentales Verfolgen nach Anspruch 1, ferner umfassend ein Ändern (444) einer zuvor bestimmten Werkzeugbahn basierend auf dem aktualisierten digitalen Modell.

4. Verfahren für segmentales Verfolgen nach Anspruch 1, ferner umfassend ein Messen (424) eines anatomischen Parameters basierend auf dem aktualisierten digitalen Modell.

5. Verfahren für segmentales Verfolgen nach Anspruch 4, ferner umfassend ein Vergleichen des gemessenen anatomischen Parameters mit einem anatomischen Zielparameter,
optional ferner umfassend das Empfangen (432) eines Operationsplans, umfassend den anatomischen Zielparameter,
ferner optional, ferner umfassend das Aktualisieren des Operationsplans basierend auf dem gemessenen anatomischen Parameter, um einen aktualisierten Operationsplan zu erbringen.

6. Verfahren für segmentales Verfolgen nach Anspruch 5, wobei der aktualisierte Operationsplan mindestens eine Operationsaufgabe zum Erreichen des anatomischen Zielparameters bei gegebenem gemessenen anatomischen Parameter umfasst.

7. Verfahren für segmentales Verfolgen nach Anspruch 1, wobei die zweiten Bilddaten einen Datenstrom umfassen und das Korrelieren und das Aktualisieren in Echtzeit oder nahezu in Echtzeit erfolgen.

8. Verfahren für segmentales Verfolgen nach Anspruch 1, ferner umfassend ein Anzeigen (420) des aktualisierten digitalen Modells auf einer Benutzerschnittstelle (110).

9. Verfahren für segmentales Verfolgen nach Anspruch 1, wobei die zweiten Bilddaten topografische Daten oder tomografische Daten umfassen.

10. System (100) für segmentales Verfolgen, umfassend:
mindestens eine Bezugsmarkierung (460);
eine Kommunikationsschnittelle (108);
eine Bildgebungsvorrichtung (112);
mindestens einen Prozessor (104); und
einen Speicher (106), der Anweisungen für Ausführung durch den mindestens einen Prozessor (104) speichert, die, wenn sie ausgeführt werden, den mindestens einen Prozessor (104) veranlassen zum:
Empfangen, über die Kommunikationsschnittstelle (108), erster Bilddaten, die einer Operationsstelle entsprechen, umfassend mindestens ein anatomisches Objekt;
Erhalten, unter Verwendung der Bildgebungsvorrichtung (112), zweiter Bilddaten, die der Operationsstelle entsprechen;
Erfassen, in den zweiten Bilddaten, einer Darstellung der mindestens einen Bezugsmarkierung (460),
Korrelieren, basierend mindestens teilweise auf der erfassten Bezugsmarkierung (460), einer Darstellung des mindestens einen anatomischen Objekts in den zweiten Bilddaten mit einer Darstellung des mindestens einen anatomischen Objekts in den ersten Bilddaten; und
Aktualisieren eines digitalen Modells des mindestens einen anatomischen Objekts basierend auf der Korrelation, **dadurch gekennzeichnet, dass** die mindestens eine Bezugsmarkierung (460) eine mindestens eine aktive Bezugsmarkierung (460) ist, wobei die mindestens eine aktive Bezugsmarkierung (460) konfiguriert ist, um elektromagnetische Wellen, die eine geeignete Frequenz für Erfassung durch einen speziellen Radar-Bildgeber aufweisen, zu erzeugen, und wobei die Bildgebungsvorrichtung (112) konfiguriert ist, um Bilddaten unter Verwendung von Radar zu erzeugen.

11. System (100) für segmentales Verfolgen nach Anspruch 10, wobei die zweiten Bilddaten einen Datenstrom umfassen, das Korrelieren während eines Empfangs der zweiten Bilddaten fortlaufend erfolgt und das Aktualisieren während des Korrelierens fortlaufend erfolgt.

12. System (100) für segmentales Verfolgen nach Anspruch 10, ferner umfassend eine Benutzerschnittstelle (110), und wobei der Speicher (106) zusätzliche Anweisungen für Ausführung durch den mindestens einen Prozessor (104) speichert, die, wenn sie ausgeführt werden, den mindestens einen Prozessor (104) ferner veranlassen zum:
Anzeigen des aktualisierten digitalen Modells auf der Benutzerschnittstelle (110).

13. System (100) für segmentales Verfolgen nach Anspruch 10, wobei der Speicher (106) zusätzliche Anweisungen für Ausführung durch den mindestens einen Prozessor (104) speichert, die, wenn sie ausgeführt werden, den mindestens einen Prozessor (104) ferner veranlassen zum:
Berechnen eines anatomischen Winkels basierend auf dem aktualisierten digitalen Modell;
und Anzeigen des berechneten anatomischen Winkels auf einer Benutzerschnittstelle (110).

14. System (100) für segmentales Verfolgen nach Anspruch 10, wobei das mindestens eine anatomische Objekt eine Vielzahl von Wirbeln umfasst.

15. System (100) für segmentales Verfolgen nach Anspruch 10, wobei die zweiten Bilddaten topografische Daten oder tomografische Daten umfassen.

## Revendications

1. Procédé de suivi segmentaire (400), comprenant :
la réception de premières données d'image correspondant à un site chirurgical comprenant au moins un objet anatomique, les premières données d'image étant générées à l'aide d'une première modalité d'imagerie ;
la réception de secondes données d'image (404) correspondant au site chirurgical, les secondes données d'image étant générées à l'aide d'une seconde modalité d'imagerie différente de la première modalité d'imagerie ;
la détection (408), dans les secondes données d'image, d'une représentation d'au moins un marqueur fiduciaire (460) ;
la corrélation (412), en fonction au moins en partie du marqueur fiduciaire détecté, d'une représentation de l'au moins un objet anatomique dans les secondes données d'image à une représentation de l'au moins un objet anatomique dans les premières données d'image ; et
la mise à jour (416) d'un modèle numérique de l'au moins un objet anatomique en fonction de la corrélation, **caractérisé en ce que**
la seconde modalité d'imagerie utilise un radar ;
l'au moins un marqueur fiduciaire est au moins un marqueur fiduciaire actif ; et
dans lequel l'au moins un marqueur fiduciaire actif est configuré pour générer des ondes électromagnétiques ayant une fréquence appropriée pour une détection par un radar imageur particulier.

2. Procédé de suivi segmentaire selon la revendication 1, comprenant en outre la génération (440) d'un guidage de navigation en fonction du modèle numérique mis à jour.

3. Procédé de suivi segmentaire selon la revendication 1, comprenant en outre la modification (444) d'une trajectoire d'outil prédéterminée en fonction du modèle numérique mis à jour.

4. Procédé de suivi segmentaire selon la revendication 1, comprenant en outre la mesure (424) d'un paramètre anatomique en fonction du modèle numérique mis à jour.

5. Procédé de suivi segmentaire selon la revendication 4, comprenant en outre la comparaison du paramètre anatomique mesuré à un paramètre anatomique cible,
éventuellement, comprenant en outre la réception (432) d'un plan chirurgical comprenant le paramètre anatomique cible,
encore éventuellement, comprenant en outre la mise à jour du plan chirurgical en fonction du paramètre anatomique mesuré afin d'obtenir un plan chirurgical mis à jour.

6. Procédé de suivi segmentaire selon la revendication 5, dans lequel le plan chirurgical mis à jour comprend au moins une tâche chirurgicale pour atteindre le paramètre anatomique cible compte tenu du paramètre anatomique mesuré.

7. Procédé de suivi segmentaire selon la revendication 1, dans lequel les secondes données d'image comprennent un flux de données, et la corrélation et la mise à jour se produisent en temps réel ou en temps quasi réel.

8. Procédé de suivi segmentaire selon la revendication 1, comprenant en outre l'affichage (420) du modèle numérique mis à jour sur une interface utilisateur (110).

9. Procédé de suivi segmentaire selon la revendication 1, dans lequel les secondes données d'image comprennent des données topographiques ou des données tomographiques.

10. Système de suivi segmentaire (100) comprenant :
au moins un marqueur fiduciaire (460) ;
une interface de communication (108) ;
un dispositif d'imagerie (112) ;
au moins un processeur (104) ; et
une mémoire (106) stockant des instructions destinées à être exécutées par l'au moins un processeur (104) qui, lorsqu'elles sont exécutées, amènent l'au moins un processeur (104) à :
recevoir, par l'intermédiaire de l'interface de communication (108), des premières données d'image correspondant à un site chirurgical comprenant au moins un objet anatomique ;
obtenir, à l'aide du dispositif d'imagerie (112), des secondes données d'image correspondant au site chirurgical ;
détecter, dans les secondes données d'image, une représentation de l'au moins un marqueur fiduciaire (460),
corréler, en fonction au moins en partie du marqueur fiduciaire détecté (460), une représentation de l'au moins un objet anatomique dans les secondes données d'image à une représentation de l'au moins un objet anatomique dans les premières données d'image ; et
mettre à jour un modèle numérique de l'au moins un objet anatomique en fonction de la corrélation, **caractérisé en ce que** l'au moins un marqueur fiduciaire (460) est au moins un marqueur fiduciaire actif (460), dans lequel l'au moins un marqueur fiduciaire actif (460) est configuré pour générer des ondes électromagnétiques ayant une fréquence appropriée pour une détection par un radar imageur particulier, et dans lequel le dispositif d'imagerie (112) est configuré pour générer des données d'image à l'aide d'un radar.

11. Système de suivi segmentaire (100) selon la revendication 10, dans lequel les secondes données d'image comprennent un flux de données, la corrélation se produit en continu pendant la réception des secondes données d'image, et la mise à jour se produit en continu pendant la corrélation.

12. Système de suivi segmentaire (100) selon la revendication 10, comprenant en outre une interface utilisateur (110), et dans lequel la mémoire (106) stocke des instructions supplémentaires destinées à être exécutées par l'au moins un processeur (104) qui, lorsqu'elles sont exécutées, amènent en outre l'au moins un processeur (104) à :
afficher le modèle numérique mis à jour sur l'interface utilisateur (110).

13. Système de suivi segmentaire (100) selon la revendication 10, dans lequel la mémoire (106) stocke des instructions supplémentaires destinées à être exécutées par l'au moins un processeur (104) qui, lorsqu'elles sont exécutées, amènent en outre l'au moins un processeur (104) à :
calculer un angle anatomique en fonction du modèle numérique mis à jour ;
et afficher l'angle anatomique calculé sur une interface utilisateur (110).

14. Système de suivi segmentaire (100) selon la revendication 10, dans lequel l'au moins un objet anatomique comprend une pluralité de vertèbres.

15. Système de suivi segmentaire (100) selon la revendication 10, dans lequel les secondes données d'image comprennent des données topographiques ou des données tomographiques.
